# EUROPEAN PATENT SPECIFICATION

(11) **EP 1 726 331 B1**
(45) Date of publication and mention of the grant of the patent: **12.11.2008**
(21) Application number: 05011470.1
(22) Date of filing: 27.05.2005
(51) Int. Cl.: A61Q 5/06, A61K 8/81

(54) **Moisture resistant hair styling composition containing two copolymers**
Wasserfeste Haarstylingzusammensetzung enthaltend zwei Copolymere
Composition de mise en forme des cheveux résistante à l'humidité comprenant deux copolymères

(43) Date of publication of application: 29.11.2006
(73) Proprietor: Wella Aktiengesellschaft, 64274 Darmstadt (DE)
(72) Inventor: Walter, Andrea, 64572 Büttelborn (DE); Birkel, Susanne, 64285 Darmstadt (DE); Franzke, Michael, 64380 Rosdorf (DE); Schmitt, Katja, 64347 Griesheim (DE)

(56) References cited:
- EP-A- 0 705 595
- US-A- 4 196 190
- US-A1- 2003 202 953
- US-B1- 6 896 878

## Description

### FIELD OF THE INVENTION

The present invention relates to a hair styling composition containing a synergistic combination of two copolymers with improved moisture resistance, humidity resistance or sweat resistance of a hair style.

### BACKGROUND OF THE INVENTION

Hair styling products are intended for helping to create individual hair styles and temporarily holding them in place for a period of time. This period of time should ideally last until the next washing of the hair with shampoo. Typical hair styling compositions are based on hair fixing polymers in a suitable carrier material, such as aqueous or aqueous-alcoholic gels containing gel forming agents. Many polymers with good hair fixing power are sensitive to moisture to the effect that they lose part or all of their hair fixing effect or get tacky when the treated hair is exhibited to moisture, e.g. in form of air humidity, rain, sweat etc. As a consequence, the desired hair style will not hold as long as wanted and starts to collapse. Therefore, it is highly desirable to improve the moisture resistance of hair styling products. At the same time, other beneficial effects such as spreadability, stylability, hold, tackiness, feel of the hair or shine of the hair should stay comparable to conventional hair styling products or should at least not be significantly reduced. Additionally, the hair styling product, although having improved resistance against water vapor or pure liquid water, should be easily removable by washing the hair with shampoo.

### SUMMARY OF THE INVENTION

The present invention is directed to a hair styling composition comprising in an aqueous or aqueous-alcoholic carrier
(A) at least one first copolymer selected from copolymers of
   (a1) at least one acidic vinyl monomer, selected from acrylic acid and methacrylic acid, or a salt thereof,
   (a2) at least one hydrophobic nonionic vinyl monomer, selected from acrylic acid esters and methacrylic acid esters,
   (a3) at least one first associative monomer and
   (a4) at least one monomer selected from the group consisting of a second associative monomer different from the first associative monomer, a semihydrophobic monomer, and a combination thereof;
      wherein the associative monomers have (i) an ethylenically unsaturated end group, (ii) a hydrophilic midsection and (iii) a hydrophobic or semi-hydrophobic end group;
(B) at least one second copolymer selected from copolymers of
   (b1) at least one C3 to C8 monoethylenically unsaturated monocarboxylic acid monomer,
   (b2) at least one nonionic vinyl monomer selected from acrylic acid alkyl esters and methacrylic acid alkyl esters, and
   (b3) at least one hydroxy substituted nonionic vinyl monomer selected from acrylic acid hydroxyalkyl esters and methacrylic acid hydroxyalkyl esters.

The present invention is further directed to methods of using the composition.

These and other features, aspects, and advantages of the present invention will become evident to those skilled in the art from a reading of the present disclosure.

### DETAILED DESCRIPTION OF THE INVENTION

The hair styling compositions of the present invention includes two different copolymers and an aqueous or aqueous-alcoholic carrier. Each of these components, as well as preferred or optional components, is described in detail hereinafter. All percentages, parts and ratios are based upon the total weight of the compositions of the present invention, unless otherwise specified. All such weights as they pertain to listed ingredients are based on the active level and, therefore, do not include solvents or byproducts that may be included in commercially available materials, unless otherwise specified. All molecular weights as used herein are weight average molecular weights expressed as grams/mole, unless otherwise specified.

Herein, "comprising" means that other steps and other ingredients which do not affect the end result can be added. This term encompasses the terms "consisting of" and "consisting essentially of". The compositions and methods/processes of the present invention can comprise, consist of, and consist essentially of the essential elements and limitations of the invention described herein, as well as any of the additional or optional ingredients, components, steps, or limitations described herein.

The term "hydrophobic monomers" as used herein, means monomers that form substantially water insoluble homopolymers. The term "hydrophilic monomers" as used herein, means monomers that form homopolymers which are substantially water soluble. The term "semihydrophobic monomers" as used herein, means monomers having two portions: (i) an ethylenically unsaturated end group portion for addition polymerization with the other monomers of the reaction mixture, and (ii) a polyoxyalkylene portion for attenuating the associations between the hydrophobic groups of the polymer or hydrophobic groups from other materials in a composition containing the polymer. A semihydrophobic monomer is similar to an associative monomer, but has a substantially non-hydrophobic end group portion. The term "associative polymer" as used herein, means polymers that contain pendant groups capable of forming associations with other groups in the polymer or other materials in the medium in which the polymer is present. Generally the pendant group has both hydrophobic and hydrophilic regions and the associations are generally based on hydrophobic interactions. According to theory, such associations result in thickening by the formation of interpolymer networks above a critical polymer overlap concentration. The terms"associative monomer" as used herein, means monomers that form associative polymers. Associative monomers generally have a polymerizable end group, a hydrophilic midsection and a hydrophobic end group. The term "polymer" as used herein shall include materials whether made by polymerization of one type of monomer (homopolymers) or made by two or more types of monomers (copolymers).

The term "water soluble" as used herein, means that the polymer is soluble in water in the present composition. In general, the polymer should be soluble at 25 °C at a concentration of 0.1 % by weight of the water solvent, preferably at 1%, more preferably at 5%, most preferably at 15%.

The terms "water resistant" and "moisture resistance" are used herein more or less synonymous, and mean a resistance against at least one of liquid water, liquids that contain water and gaseous water, i.e. water vapor and air humidity. The term "humidity resistant" as used herein, means a resistance specifically against gaseous water, i.e. water vapor and air humidity.

All cited references are incorporated herein by reference in their entireties. Citation of any reference is not an admission regarding any determination as to its availability as prior art to the claimed invention.

### First copolymer

The first copolymer as defined by claim 1 is a copolymer of (a1) at least one acidic vinyl monomer, (a2) at least one hydrophobic nonionic vinyl monomer, (a3) at least one first associative monomer and (a4) at least one monomer selected from the group consisting of a second associative monomer different from the first associative monomer, a semihydrophobic monomer, and a combination thereof. The first copolymer of the present invention is preferably used in an amount of from 0,1 to 15% by weight, more preferably from 0,5 to 10% by weight or 1 to 5% by weight.

The acidic vinyl monomer preferably comprises about 10 to about 75% by weight of the total monomer mixture, more preferably about 25 to about 65% by weight, and most preferably about 30 to about 60% by weight. Acidic vinyl monomers suitable for use in the present invention are acrylic and methacrylic acid, or a salt thereof.

The nonionic vinyl monomer preferably comprises about 10 to about 90% by weight of the total monomer mixture, more preferably about 25 to about 75% by weight, and most preferably about 30 to about 60% by weight. Nonionic vinyl monomers suitable for use in the present invention are acrylic and methacrylic acid esters such as C₁-C₁₈ alkyl esters of acrylic acid and of methacrylic acid, methacrylamidoethyl-N-ethylene urea, and combinations thereof. Particularly preferred nonionic vinyl monomers are acrylic acid C1- to C4-alkyl esters and methacrylic acid C1-to C4-alkyl esters such as ethylacrylate or methylmethacrylate.

Preferably, the associative monomers in the monomer mixture independently comprise about 0.1 to about 25% by weight of the monomer mixture, more preferably about 0.25 to about 20% by weight, most preferably about 0.5 to about 15% by weight. Associative monomers suitable for use in the present invention are compounds having an ethylenically unsaturated end group portion (i) for polymerization with the other monomers of the system; a polyoxyalkylene midsection portion (ii) for imparting selective hydrophilic properties to the product polymer and a hydrophobic end group portion (iii) for providing selective hydrophobic properties to the polymer. The portion (i) supplying the ethylenically unsaturated end group preferably is derived from an α,β-ethylenically unsaturated mono or di-carboxylic acid or the anhydride thereof, more preferably a C₃ or C₄ mono- or di-carboxylic acid or the anhydride thereof. Alternatively, portion (i) of the associative monomer can be derived from an allyl ether or vinyl ether; a nonionic vinyl-substituted urethane monomer; or a vinyl-substituted urea reaction product. The midsection portion (ii) is preferably a polyoxyalkylene segment of about 5 to about 250, more preferably about 10 to about 120, and most preferably about 15 to about 60 repeating C₂-C₇ alkylene oxide units. Preferred midsection portions (ii) include polyoxyethylene, polyoxypropylene, and polyoxybutylene segments comprising about 5 to about 150, more preferably about 10 to about 100, and most preferably about 15 to about 60 ethylene, propylene or butylene oxide units, and random or non-random sequences of ethylene oxide, propylene oxide and or butylene oxide units. The hydrophobic end group portion (iii) of the associative monomers is preferably a hydrocarbon moiety belonging to one of the following hydrocarbon classes: a C₈-C₄₀ linear alkyl, an aryl-substituted C₂-C₄₀ alkyl, a C₂-C₄₀ alkyl-substituted phenyl, a C₈-C₄₀ branched alkyl, a C₈-C₄₀ carbocyclic alkyl; and a C₈-C₈₀ complex ester. Non-limiting examples of suitable hydrophobic end group portions (iii) of the associative monomers are linear or branched alkyl groups having about 8 to about 40 carbon atoms, such as capryl (C₈), iso-octyl (branched C₈), decyl (C₁₀), lauryl (C₁₂), myristyl (C₁₄), cetyl (C₁₆), cetearyl (C₁₆-C₁₈), stearyl (C₁₈), isostearyl (branched C₁₈), arachidyl (C₂₀), behenyl (C₂₂), lignoceryl (C₂₄), cerotyl (C₂₆), montanyl (C₂₈), melissyl (C₃₀), lacceryl (C₃₂), and the like. Examples of linear and branched alkyl groups having about 8 to about 40 carbon atoms that are derived from a natural source include, without being limited thereto, alkyl groups derived from hydrogenated peanut oil, soybean oil and canola oil (all predominately C₁₈), hydrogenated tallow oil (C₁₆-C₁₈), and the like; and hydrogenated C₁₀-C₃₀terpenols, such as hydrogenated geraniol (branched C₁₀), hydrogenated farnesol (branched C₁₅), hydrogenated phytol (branched C₂₀), and the like. Non-limiting examples of suitable C₂₋C₄₀ alkyl-substituted phenyl groups include octylphenyl, nonylphenyl, decylphenyl, dodecylphenyl, hexadecylphenyl, octadecylphenyl, isooctylphenyl, sec-butylphenyl, and the like. Suitable C₈-C₄₀ carbocylic alkyl groups include, without being limited thereto, groups derived from sterols from animal sources, such as cholesterol, lanosterol, 7-dehydrocholesterol, and the like; from vegetable sources, such as phytosterol, stigmasterol, campesterol, and the like; and from yeast sources, such as ergosterol, mycosterol, and the like. Other carbocyclic alkyl hydrophobic end groups useful in the present invention include, without being limited thereto, cyclooctyl, cyclododecyl, adamantyl, decahydronaphthyl, and groups derived from natural carbocyclic materials, such as pinene, hydrogenated retinol, camphor, isobornyl alcohol, and the like. Exemplary aryl-substituted C₂-C₄₀ alkyl groups include, without limitation thereto, styryl (e.g., 2-phenylethyl), distyryl (e.g., 2,4-diphenylbutyl), tristyryl (e.g., 2,4,6-triphenylhexyl), 4-phenylbutyl, 2-methyl-2-phenylethyl, tristyrylphenolyl, and the like. Non-limiting examples of suitable C₈-C₈₀ complex esters include hydrogenated castor oil (predominately the triglyceride of 12-hydroxystearic acid); 1,2-diacyl glycerols, such as 1,2-distearyl glycerol, 1,2-dipalmityl glycerol, 1,2-dimyristyl glycerol, and the like; di-, tri-, or poly-esters of sugars, such as 3,4,6-tristearyl glucose, 2,3-dilauryl fructose, and the like; and sorbitan esters. Useful associative monomers can be prepared by any method known in the art.

Particularly preferred associative monomers include cetyl polyethoxylated methacrylate (CEM), cetearyl polyethoxylated methacrylate (CSEM), stearyl polyethoxylated (meth)acrylate, arachidyl polyethoxylated (meth)acrylate, behenyl polyethoxylated methacrylate (BEM), cerotyl polyethoxylated (meth)acrylate, montanyl polyethoxylated (meth)acrylate, melissyl polyethoxylated (meth)acrylate, lacceryl polyethoxylated (meth)acrylate, tristyrylphenol polyethoxylated methacrylate (TEM), hydrogenated castor oil polyethoxylated methacrylate (HCOEM), canola polyethoxylated (meth)acrylate, and cholesterol polyethoxylated methacrylate (CHEM), where the polyethoxylated portion of the monomer comprises about 5 to about 100, preferably about 10 to about 80, and more preferably about 15 to about 60 ethylene oxide repeating units.

When the copolymer is made of two different associative monomers, the two hydrophobic end groups of the two associative monomers are each independently selected from the same or different hydrocarbon classes, and when the two hydrophobic end groups are selected from the same hydrocarbon class, the molecular formulas of the hydrophobic end groups differ preferably by at least about 8 carbon atoms.

When utilized, semihydrophobic monomers preferably comprise about 0,1 to about 25% by weight of the total monomer mixture, more preferably about 0,5 to about 20% by weight, and most preferably about 1 to about 15% by weight. Semihydrophobic monomers suitable for use in the present invention are monomers having two portions: (i) an ethylenically unsaturated end group portion for addition polymerization with the other monomers of the reaction mixture, and (ii) a polyoxyalkylene portion as a substantially non-hydrophobic end group portion. The unsaturated end group-portion (i) supplying the vinyl or other ethylenically unsaturated end group for addition polymerization is preferably derived from an allyl ether, a vinyl ether, a nonionic urethane monomer, an α,β-ethylenically unsaturated mono or di-carboxylic acid or the anhydride thereof, preferably a C₃ or C₄ mono- or di-carboxylic acid, or the anhydride thereof. The polymerizable unsaturated end group portion (i) can also be derived from a C₈-C₃₀ unsaturated fatty acid group containing at least one free carboxy-functional group. This C₈-C₃₀ group is part of the unsaturated end group portion (i) and is different from the hydrophobic groups pendant to the associative monomers, which are specifically separated from the unsaturated end group of the associative monomer by a hydrophilic "spacer" portion. The polyoxyalkylene portion (ii) specifically comprises a long-chain polyoxyalkylene segment, which is substantially similar to the hydrophilic portion of the associative monomers. Preferred polyoxyalkylene portions (ii) include polyoxyethylene, polyoxypropylene, and polyoxybutylene units comprising about 2 to about 250, and preferably about 10 to about 100 ethylene oxide, propylene oxide, or butylene oxide units, or random or non-random sequences of ethylene oxide, propylene oxide, and/or butylene oxide units.

Preferred semihydrophobic monomers include those of the following formulas:

R¹CH=CR¹-A-(CH₂)ₚ-(O)ᵣ-(R³O)ᵥ-R⁴

or

D-A-(CH₂)ₚ-(O)ᵣ-(R³O)ᵥ-R⁴

wherein, in each of the formulas, each R¹ is independently H, C₁-C₃₀ alkyl, -C(O)OH, or -C(O)OR²; R² is C₁-C₃₀ alkyl; A is -CH₂C(O)O- , -C(O)O-, -O-, -CH₂O-, -NHC(O)NH-, -C(O)NH-. -Ar-(CE₂)_{z} NHC(O)O-, -Ar-(CE₂)_{z}-NHC(O)NH-, or -CH₂CH₂NHC(O)-; Ar is a divalent aryl; E is H or methyl; z is 0 or 1; p is an integer in the range of 0 to about 30, and r is 0 or 1, with the proviso that when p is 0, r is 0, and when p is in the range of 1 to about 30, r is 1; (R³O)ᵥ is a polyoxyalkylene, which is a homopolymer, a random copolymer or a block copolymer of C₂-C₄ oxyalkylene units, wherein R³ is C₂H₄, C₃H₆, C₄H₈, and v is an integer in the range of about 5 to about 250, , preferably about 5 to about 100, more preferably about 10 to about 80, and most preferably about 15 to about 60; R⁴ is H or C₁-C₄ alkyl; and D is a C₈-C₃₀unsaturated alkyl or a carboxy-substituted C₈-C₃₀ unsaturated alkyl.

Particularly preferred semihydrophobic monomers include monomers having the following chemical formulas:

CH₂=CH―O―(CH₂)ₐ―O―(C₃H₆O)_{b}―(C₂H₄O)_{c}―H

or

CH₂=CH―CH₂―O―(C₃H₆O)_{d}―(C₂H₄O)ₑ―H;

wherein a is 2, 3, or 4; b is an integer in the range of 1 to about 10; more preferably about 2 to about 8, most preferably about 3 to about 7; c is an integer in the range of about 5 to about 50, more preferably about 8 to about 40, most preferably about 10 to about 30; d is an integer in the range of 1 to about 10, more preferably about 2 to about 8, most preferably about 3 to about 7; and e is an integer in the range of about 5 to about 50, more preferably about 8 to about 40. Examples of preferred semihydrophobic monomers include polymerizable emulsifiers commercially available under the trade names EMULSOGEN® R109, R208, R307, RAL109, RAL208, and RAL307 sold by Clariant Corporation; BX-AA-E5P5 sold by Bimax, Inc.; and MAXEMUL® 5010 and 5011 sold by Uniqema; and combinations thereof. Particularly preferred semihydrophobic monomers include EMULSOGEN® R109, R208, and R307, BX-AA-E5P5, MAXEMUL® 5010 and 5011, and combinations thereof. According to the manufacturers: EMULSOGEN® R109 is a randomly ethoxylated/propoxylated 1,4-butanediol vinyl ether having the empirical formula CH₂=CH―O(CH₂)₄O(C₃H₆O)₄(C₂H₄O)₁₀H; EMULSOGEN® R208 is a randomly ethoxylated/propoxylated 1,4-butanediol vinyl ether having the empirical formula CH₂=CH-O(CH₂)₄O(C₃H₆O)₄-(C₂H₄O)₂₀H; EMULSOGEN® R307 is a randomly ethoxylated/propoxylated 1,4-butanediol vinyl ether having the empirical formula CH₂=CH-O(CH₂)₄O(C₃H₆O)₄-(C₂H₄O)₃₀H; EMULSOGEN® RAL109 is a randomly ethoxylated/propoxylated allyl ether having the empirical formula CH₂=CHCH₂O-(C₃H₆O)₄(C₂H₄O)₁₀H; EMULSOGEN® RAL208 is a randomly ethoxylated/propoxylated allyl ether having the empirical formula CH₂CHCH₂O(C₃H₆O)₄(C₂H₄O)₂₀H; EMULSOGEN® RAL307 is a randomly ethoxylated/propoxylated allyl ether having the empirical formula CH₂CHCH₂O(C₃H₆O)₄(C₂H₄O)₃₀H; MAXEMUL® 5010 is a carboxy-functional C₁₂-C₁₅ alkenyl hydrophobe, ethoxylated with about 24 ethylene oxide units; MAXEMUL® 5011 is a carboxy-functional C₁₂-C₁₅alkenyl hydrophobe, ethoxylated with about 34 ethylene oxide units; and BX-AA-E5P5 is a randomly ethoxylated/ propoxylated allyl ether having the empirical formula CH₂=CHCH₂O(C₃H₆O)₅(C₂H₄O)₅H.

The copolymers suitable as first copolymers of the present invention can optionally be made from at least one additional compound selected from the group consisting of crosslinking agents, chain transfer agents, and a combination thereof. These agents are well known in the art. Suitable crosslinking agents and chain transfer agents are for example described in US 2003/0207988 A1 paragraphs [0088] to [0100]. Preferred cross linking agents are acrylate and methacrylate esters of polyols having at least two acrylate or methacrylate ester groups, such as trimethylolpropane triacrylate, trimethylolpropane dimethacrylate, polyethylene glycol dimethacrylate, ethoxylated bisphenol A dimethacrylate, and the like. Preferred chain transfer agents are selected from a variety of thio and disulfide containing compounds, such as C₁-C₁₈ alkyl mercaptans, mercaptocarboxylic acids, mercaptocarboxylic esters, thioesters, C₁-C₁₈ alkyl disulfides, aryldisulfides, polyfunctional thiols, and the like; phosphites and hypophosphites; haloalkyl compounds, such as carbon tetrachloride, bromotrichloromethane, and the like; and unsaturated chain transfer agents, such as alpha-methylstyrene.

Preferred copolymers suitable as first copolymers of the present invention are those described in US 2003/0207988 A1. A suitable copolymer has the INCI-name polyacrylate-14 and is marketed under the trade name FIXATE™ PLUS by Noveon, Inc..

### Second copolymer

The second copolymer as defined by claim 1 is a copolymer of (b1) at least one C3 to C8 monoethylenically unsaturated monocarboxylic acid monomer, (b2) at least one nonionic vinyl monomer selected from acrylic acid alkyl esters and methacrylic acid alkyl esters, and (b3) at least one hydroxy substituted nonionic vinyl monomer selected from acrylic acid hydroxyalkyl esters and methacrylic acid hydroxyalkyl esters. The second copolymer of the present invention is preferably used in an amount of from 0,1 to 15% by weight, more preferably from 0,4 to 10% by weight or 1 to 5% by weight.

The C3 to C8 monoethylenically unsaturated monocarboxylic acid monomer (b1) preferably comprises about 2 to about 50% by weight of the total monomer mixture, more preferably about 2 to about 30% by weight, and most preferably about 12 to about 26% by weight. The C₃ to C₈ monoethylenically unsaturated monocarboxylic acid is preferably (meth)acrylic acid, crotonic acid, or combinations thereof. More preferably, the C₃ to C₈ monoethylenically unsaturated monocarboxylic acid is methacrylic acid.

The nonionic vinyl monomer (b2) preferably comprises about 5 to about 95% by weight of the total monomer mixture, more preferably about 45 to about 90% by weight, and most preferably about 70 to about 80% by weight. The C1 to C8 alkyl groups of the nonionic vinyl monomer (b2) can be linear or branched. Preferably, the alkyl group is a C₁ to C₅ alkyl alkyl group such as for example, methyl, ethyl, propyl, butyl, or pentyl or combinations thereof. More preferably the nonionic vinyl monomer (b2) comprises at least one first monomer selected from C₁ to C₃ alkyl methacrylates and at least one second monomer selected from C₂ to C₅ alkyl acrylates. Most preferably the nonionic vinyl monomer (b2) comprises a first monomer which is methyl methacrylate and a second monomer which is butyl acrylate, e.g. n-butyl acrylate. The amount of the at least one C₁ to C₃ alkyl methacrylate in the hair fixative resin is preferably from 5 to 71, more preferably from 41 to 60% by weight based on the total monomers used to form the hair fixative resin. The amount of the C₂ to C₅ alkyl acrylate is preferably from 2 to 67 and more preferably from 10 to 30% by weight, based on the total monomer used to form the acrylic hair fixative resin.

The hydroxy substituted nonionic vinyl monomer (b3) preferably comprises about 2 to about 70% by weight of the total monomer mixture, more preferably about 2 to about 26% by weight, and most preferably about 5 to about 20% by weight. The alkyl group of the hydroxy substituted nonionic vinyl monomer (b3) is preferably a C₁ to C₅ alkyl group. For example the hydroxy substituted nonionic vinyl monomer (b3) is preferably hydroxymethyl acrylate, hydroxyethyl acrylate, hydroxypropyl acrylate, hydroxybutyl acrylate, hydroxypentyl acrylate, hydroxymethyl methacrylate, hydroxyethyl methacrylate, hydroxypropyl methacrylate, hydroxybutyl methacrylate, hydroxypentyl methacrylate, or combinations thereof. More preferably the hydroxy substituted nonionic vinyl monomer (b3) is hydroxyethyl methacrylate or hydroxypropyl acrylate or combinations thereof, most preferably hydroxyethyl methacrylate.

The second copolymer preferably has a weight average molecular weight (Mw) from 25000 to 250000, more preferably from 50000 to 150000, as measured by gel permeation chromatography using a 100,000 Mw methylmethacrylate polymer as the standard. The second copolymer preferably has a glass transition temperature (Tg) above room temperature (25°C), more preferably from 40 to 120 °C, and most preferably from 50 to 100 °C. Preferred copolymers suitable as second copolymers of the present invention are those described in U.S. pat. no. 4,196,190, EP 0 705 595 A2 and EP 1 142 554 A1. A suitable copolymer has the INCI-name acrylates/hydroxyesters acrylates copolymer and is marketed under the trade name ACUDYNE™ 180 by Rohm and Haas Company.

### Carrier

The carrier of the hair styling composition according to the invention can be aqueous of aqueous-alcoholic. By "aqueous" it is meant that the compositions contain almost only water as solvant, i.e. organic solvants such as C1- to C4 alcohols are not present or they are present only in very minor amounts such as below 2 or below 1% by weight of the total composition. Deionized water is preferably used. Water from natural sources containing mineral cations can also be used, depending on the desired characteristic of the product. By "aqueous-alcoholic" it is meant that the compositions contain significant amounts of water as well as significant amounts of alcoholic solvants. Significant amounts are amounts of e.g. at least 5% by weight or more. The level and species of the carrier are selected according to the compatibility with other components, and other desired characteristics of the product. Alcoholic solvants are organic compounds which are liquid at room temperature (25°). The amount of alcohol is preferably 0 to 50% by weight, more preferably from 1 to 25% by weight of the total composition. Alcohols can be those conventionally used for cosmetic purposes, e.g. monohydric C1 to C6 alcohols such as ethanol and isopropanol. Ethanol is especially preferred. The water content is prerferably from 40 to 95, more preferred from 50 to 90% by weight. An aqueous-alcoholic carrier can contain for example 5 to 25% by weight ethanol and 60 to 80% by weight water, based on the total composition. The pH is preferably in the range of from 6 to 9, more prefarably from 6,5 to 8. Buffers and other pH adjusting agents can be included to achieve or stabilise the desirable pH.

### Polyhydric alcohols

In one embodiment of the invention, the hair styling composition additionally contains at least one polyhydric alcohol for further improving the hair shine. The polyhydric alcohols have at least two alcoholic hydroxyl groups. They have preferably 2 to 6 carbon atoms and 2 to 6 hydroxyl groups such as glycerol, C2- to C4-alkylenglycols, and sorbitol. Especially preferred are glycerol and C2- to C4-alkylenglycols, such as ethylenglycol and propylenglycol. The amount of polyhydric alcohol is preferably from 0,1 to 15, more preferably from 0,5 to 6% by weight based on the total composition.

### Gel forming agents

In one embodiment of the invention, the hair styling composition has the form of a gel and additionally contains at least one gel forming agent. The amount of gel forming agents is preferably from 0,05 to 30, more preferably from 0,2 to 20 and most preferably from 0,5 to 10% by weight based on the total composition. Suitable gel forming agents are for example one or a mixture of:
- synthetic polymer such as e.g. crosslinked polyacrylates;
- polymers on a natural basis, e.g. based on sclerotium gum; starch; gelatine; cellulose and cellulose derivatives such as carboxymethyl cellulose,hydroxyalkyl cellulose such as hydroxypropylcellulose or hydroxyethylcellulose, methylcelluose or hydroxyproyplmethylcellulose; microcrystalline cellulose; agar-agar; carrageenan, alginates, carouba gum, guar and guar derivatives such as alkylated and hydroxyalkylated guar; karaya gum; xanthan gum; dehydroxanthan; gum arabicum, pektin
- inorganic thickeners, e.g. hectorite, bentonite, metal silicates such as aluminium silicates or magnesium silicates.

In particular, gel forming agents are:
copolymers of at least one first monomer selected from acrylic acid and methacrylic acid and at least one second monomer selected from esters of acrylic acid and ethoxylated fatty alcohols; crosslinked polyacrylic acid; crosslinked copolymers of at least one first monomer selected from acrylic acid and methacrylic acid and at least one second monomer selected from esters of acrylic acid and C10 to C30 alcohols such as those with INCI-name Acrylates/C10-30 Alkyl Acrylate Crosspolymer having tradenames Pemulen™ TR-1, Pemulen™ TR-2, Carbopol™ 1342, Carbopol™ 1382, and Carbopol™ ETD 2020, all available from Noveon, Inc.; copolymers of at least one first monomer selected from acrylic acid and methacrylic acid and at least one second monomer selected from esters of itaconic acid and ethoxylated fatty alcohols; copolymers of at least one first monomer selected from acrylic acid and methacrylic acid and at least one second monomer selected from esters of itaconic acid and ethoxylated C10 to C30 alcohols and at least one third monomer selected from amino C 1 to C4-alkylacrylates; copolymers of two or more monomers selected from from acrylic acid, methacrylic acid, acrylic acid esters and methacrylic acid esters; copolymers of vinylpyrrolidone and ammonium acryloyl dimethyltaurate; copolymers of ammonium acryloyl dimethyltaurate and at least one monomer selected from esters of methacrylic acid and ethoxylated fatty alcohols; hydroxyethyl cellulose; hydroxypropyl cellulose; hydroxypropyl guar; glyceryl polyacrylate; glyceryl polymethacrylate; copolymers of styrene and at least one C2, C3 or C4-alkylene; gel forming polyurethanes; hydroxypropyl starch phosphate; polyacrylamide; copolymer of maleic acid anhydride and methylvinylether crosslinked wich decadiene; carob bean gum; guar gum; xanthan; dehydroxanthan; carrageenan; karaya gum; hydrolysed corn starch; copolymers of polyethylenoxide, fatty alcohols und saturated methylene diphenyldiisocyanate (e.g. PEG-150/stearyl alcohol/ SMDI copolymer).

### Additional hair fixing polymers

In one embodiment of the invention, the hair styling composition additionally contains at least one additional hair fixing polymer, different from the first and second copolymer. The amount of additional hair fixing polymer is preferably from 0,01 bis 30, more preferably from 0,1 bis 15, and most preferred from 0,5 bis 10% by weight based on the total composition. The hair fixing polymer can be nonionic, anionic, cationic amphoteric or zwitterionic, preferably it is nonionic or anionic. The hair fixing polymer can be synthetic or natural. The term "natural polymer" also comprises chemically modified polymers of natural origin. Preferred are polymers which are soluble in the aqueous or aqueous-alcoholic carrier. Hair fixing polymers are polymeric compounds which impart hair-holding or style-retention properties to hair, e.g. when applied as 0,01 to 5% by weight aqueous, alcoholic or aqueous-alcoholic solution or dispersion. In particular, hair fixing polymers are those polymers listed in the International Cosmetic Ingredient Dictionary and Handbook, 10^{th} edition 2004 with the function "Hair Fixatives":

Suitable synthetic, non-ionic hair fixing polymers are for example:
homo- oder copolymers of at least one monomer selected from vinyl pyrrolidone; vinyl caprolactam; vinyl ester e.g. vinyl acetate, vinyl alcohol, acrylamide, methacrylamide, alkyl- and dialkyl acrylamide, alkyl- und dialkyl methacrylamide, dialkylaminoalkyl methacrylamide, dialkylaminoalkyl acrylamide, alkylacrylate, alkylmethacrylate, propylenglycol oder ethylenglykol, wherein preferred alkyl groups of these monomers are C1- to C7-alkyl groups, more preferred C1- to C3-alkyl groups. Suitable are e.g. homopolymers of vinyl caprolactam, homopolymers of vinyl pyrrolidone, homopolymers of N-vinyl formamide. Suitable hair fixing polymers are also copolymers of vinyl pyrrolidone and vinyl acetate; terpolymers of vinyl pyrrolidone, vinyl acetate and vinyl propionate; terpolymers of vinyl pyrrolidone, vinyl caprolactam and dialkylaminoalkyl (meth)acrylate; terpolymers of vinyl pyrrolidone, vinyl caprolactam and dialkylaminoalkyl (meth)acrylamide; polyacrylamide; polyvinyl alcohol; and hair fixing polyethylen glycol/polypropylen glycol copolymers. Preferred are nonionic vinyl lactam homo- or copolymers. Suitable vinyl lactams are e.g. vinyl caprolactam and vinylpyrrolidone. Especially preferred are polyvinyl pyrrolidone, polyvinyl caprolactam and polyvinyl pyrrolidone/vinyl acetate copolymers which are marketed e.g as Luviskol® VA 37 und Luviskol® VA 64.

Suitable synthetic, anionic hair fixing polymers can be synthetic or natural homo- or copolymers from monomeric units with acid groups. The monomers with acid groups can be copolymerised with monomers without acid groups. Preferred acid groups are - COOH, -SO₃H, -OSO₃H, -OPO₂H und -OPO₃H₂, carboxylic acid being most preferred. The acid groups can be unneutralised, partially neutralised or completely neutralised. Preferred is a degree of the anionic, neutralised form of from 50 to 100%. Suitable monomers are ethylenically unsaturated, radically polymerisable compounds carrying at least one acid group, e.g. styrene sulfonic acid, 2-acrylamide-2-methylpropane sulfonic acid or carboxyvinyl monomers like acrylic acid, methacrylic acid, crotonic acid, fumaric acid, maleic acid, maleic anhydride and its monoesters or itaconic acid.

Comonomers without acid groups are e.g. acrylamide, methacrylamide, alkyl- and dialkyl acrylamide, alkyl- and dialkyl methacrylamide, alkylacrylate, alkylmethacrylate, vinyl caprolactone, vinyl pyrrolidone, vinyl ester, vinyl alcohol, propylen glycol or ethylen glycol, amine substituted vinyl monomers such as dialkylaminoalkyl acrylate, dialkylaminoalkyl methacrylate, monoalkylaminoalkyl acrylate and monoalkylaminoalkyl methacrylate, wherein preferred alkyl groups are C1- to C7-alkyl groups, especially C1- to C3-alkyl groups.

Suitable anionic hair fixing polymers are in particular copolymers of acrylic or methacrylic acid with monomers selected from acrylic acid esters, methacrylic acid esters, acrylamides, methacrylamides and vinyl pyrrolidone; homopolymers of crotonic acid; copolymers of crotonic acid with monomers selected from vinyl esters, acrylic acid esters, methacrylic acid esters, acrylamides, methacrylamides. A natural anionic hair fixing polymer is shellac. Preferred anionic hair fixing polymers are vinylacetate/crotonic acid copolymer; partially esterified copolymers of vinyl methylether and maleic anhydride; terpolymers of acrylic acid, alkyl acrylate and N-alkyl acrylamide, e.g. acrylic acid/ethyl acrylate/N-t-butyl acrylamide terpolymer; terpolymers of vinyl acetate, crotonic acid and vinyl alkanoate, e.g. vinyl acetate/crotonic acid/vinyl neodecanoate copolymer.

Suitable synthetic, amphoteric hair fixing polymers are polymers with anionic or acidic functional groups as well as cationic or basic functional groups. The acidic or anionic functional groups are those as definied above for the anionic polymers. Cationic or basic functional groups are in particular primary, secondary or tertiary amine groups or quaternary ammonium groups. Preferred examples are copolymers of alkyl acrylamide (especially octyl acrylamide), alkylaminoalkyl methacrylate (especially t-butylaminoethyl methacrylate) and two or more monomers selected from acrylic acid, methacrylic acid and their esters, wherein the alkylgroups have from 1 to 4 C-atoms and at least one of the monomers has an acid group. A marketed product is e.g. Amphomer^{®} oder Amphomer^{®} LV-71 of National Starch. Further examples for hair fixing polymers are copolymers of acrylic acid, methyl acrylate and methacrylamidopropyl trimethylammonium chloride (INCI-name: polyquatemium-47); copolymer of acrylamidopropyl trimethylammonium chloride and acrylates; or copolymers of acrylamide, acrylamidopropyl trimethylammonium chloride, 2-amidopropyl acrylamide sulfonate and dimethylaminopropyl amine (INCI-name: polyquaternium-43). Suitable are also polymers with betaine groups, e.g. copolymers of methacryloyl ethylbetaine and two or more monomers selected from acrylic acid and its alkyl esters (INCI-name Methacryloyl Ethyl Betaine/Acrylates Copolymer).

Suitable cationic hair fixing polymers are polymers with cationic or basic functional groups. Cationic or basic functional groups are in particular primary, secondary or tertiary amine groups or quaternary ammonium groups. The cationic charge density is preferably from 1 to 7 meq/g. The cationic polymers can be homopolymers or copolymers wherein the cationic or basic functional group can be part of the polymeric backbone or can be a pendant group. Monomers with cationic or basic groups can be copolymerised with monomers without cationic or basic group.

Suitable cationic monomers are ethylenically unsaturated radically polymerisable compounds with at least one cationic or basic group, e.g. ammonium substituted vinyl monomers such as trialkyl methacryloxy alkylammonium, trialkyl acryloxy alkyl ammonium, dialkyl diallyl ammonium and quaternary vinyl ammonium monomers with cyclic nitrogen containing groups such as pyridinium, imidazolium or quaternary pyrrolidones, e.g. alkylvinyl imidazolium, alkylvinyl pyridinium. The alkyl groups of these monomers are preferably lower alkyl groups such as C1 to C7 alkyl groups, more preferred C1 to C3 alkyl groups. The cationic monomers can be polymerised with non-cationic comonomers. Non-cationic comonomers are e.g. acrylamide, methacrylamide, alkyl- and dialkyl acrylamide, alkyl- and dialkyl methacrylamide, alkylacrylate, alkylmethacrylate, vinyl caprolactone, vinyl pyrrolidone, vinyl ester such as vinyl acetate, vinyl alcohol, propylen glycol or ethylen glycol, wherein preferred alkyl groups are C1- to C7-alkyl groups, especially C1- to C3-alkyl groups.

Suitable cationic hair fixing polymers are for examples those listed in the International Cosmetic Ingredient Dictionary and Handbook as polyquaternium, e.g. methylvinyl imidazolium chloride/vinyl pyrrolidone copolymer (Polyquaternium-16) or quaternised vinyl pyrrolidone/dimethylaminoethyl methacrylate copolymer (Polyquaternium-11). Preferred synthetic cationic hair fixing polymers are: poly(dimethyl diallyl ammonium chloride); copolymers of acrylamide and dimethyl diallyl ammonium chloride; quaternary ammonium polymers made by reaction of diethylsulfate and a copolymer of vinyl pyrrolidone and dimethylaminoethyl methacrylate, such as vinyl pyrrolidone/dimethylaminoethyl methacrylate methosulfate copolymer (e.g. GAFQUAT^{®} 755 N, GAFQUAT^{®} 734); quaternary ammonium polymers of methylvinyl imidazolium chloride and vinyl pyrrolidone (e.g. LUVIQUAT^{®} HM 550); Polyquaternium-35; Polyquaternium-57; polymer of trimethylammoniumethyl methacrylate chloride; terpolymer of dimethyl diallyl ammonium chloride, sodium acrylate and acrylamide (e.g. MERQUAT^{®} Plus 3300); copolymer of vinyl pyrrolidone, dimethylaminopropyl methacrylamide and methacryloyl aminopropyllauryl dimethyl ammonium chloride; terpolymer of vinylpyrrolidone, dimethylaminoethyl methacrylate and vinyl caprolactam (e.g. GAFFIX^{®} VC 713); vinyl pyrrolidone / methacryl amidopropyl trimethylammonium chloride copolymer (e.g. GAFQUAT^{®} HS 100); copolymer of vinyl pyrrolidone and dimethylaminoethyl methacrylate; copolymer of vinyl pyrrolidone, vinyl caprolactam and dimethylaminopropyl acrylamide; poly- or oligoester made of at least one monomer selected from hydroxyacids which are substituted with at least one quaternary ammonium group.

Sutitable cationic polymers derived from natural polymers are for example cationic derivatives of polysaccharides such as cationic derivatives of cellulose, starch or guar. Suitable are also chitosan and chitosan derivatives. Cationic polysaccharides have for example the general formula

G-O-B-N⁺R^{a}R^{b}R^{c} X⁻

Wherein G is an anhydroglucose group such as starch anhydroglucose or cellulose anhydroglucose; B is a divalent bridging group such as alkylen, oxyalkylen, polyoxyalkylen or hydroxyalkylen; R^{a}, R^{b} and R^{c} are independent from one another alkyl, aryl, alkylaryl, arylalkyl, alkoxyalkyl or alkoxyaryl with each up to 18 carbon atoms, wherein the total number of carbon atoms in R^{a}, R^{b} and R^{c} is preferably a maximum of 20; X is a counter ion, such as halogen, acetate, phosphate, nitrate or alkylsulfate, preferably chloride. Cationic cellulose polymers are for example those with the INCI-names Polyquaternium-10 or Polyquaternium-24. A cationic guar derivative is for example that with the INCI-name Guar Hydroxypropyltrimonium Chloride.

Especially preferred cationic hair fixing polymers are chitosan, chitosan salts and chitosan derivatives. Chitosans are totally or partially deacetylated chitines. The molecular weight can be for example from about 20000 to about 5 Millionen g/mol, e.g.. from 30000 to 70000 g/mol for lower molecular weight chitosan. Preferred are high molecular chitosans with a molecular weight above 100000 g/mol, more preferred from 200000 to 700000 g/mol. The degree of deacetylation is preferably from 10 to 99%, more preferred from 60 to 99%. A preferred chitosan salt is chitosonium pyrrolidonecarboxylate, e.g. KYTAMER® PC with a molecular weight of about 200000 to 300000 g/mol and a degree of deacetylation of 70 bis 85%. Chitosan derivatives are for example quaternised chitosans, alkylated chitsoans or hydroxyalkylated chitsoans such as hydroxyethyl-, hydroxypropyl- or hydroxybutyl chitosan. The chitosan or chitosan derivatives are preferably partially or completely neutralised. The degree of neutralisation is preferably at least 50%, more preferred from 70 und 100%, based on the total number of amino groups. In principle, all cosmetic acceptable inorganic or organic acids can be used for neutralisation, such as formic acid, tartaric acid, malic acid, lactic acid, citric acid, pyrrolidone carboxylic acid, glycolic acid, hydrochloric acid etc., pyrrolidone carboxylic acid being especially preferred.

Preferred cationic polymers on a natural basis are:
cationic cellulose derivatives made from hydroxyethylcellulose and diallyl dimethyl ammonium chloride; cationic cellulose derivatives made from hydroxyethylcellulose and trimethyl ammonium substituted epoxide; chitosan and ist salts; hydroxyalkyl chitosan and ist salts; alkylhydroxyalkyl chitosan and ist salts; N-hydroxyalkyl chitosan alkylether.

Most preferred hair fixing polymers are polyvinylpyrrolidone (INCI-name PVP; trade names e.g. Luviskol™ K30, K85, K90 available from BASF); copolymers of vinylpyyrolidone and vinylacetate (INCI-name VP/VA copolymer; trade names e.g. Luviskol™ VA37, VA64 available from BASF); copolymers of vinylpyrrolidone, methacrylamide and vinylimidazole (INCI-name VP/Methacrylamide/Vinyl Imidazole Copolymer, trade name Luviset™ Clear available from BASF); copolymers of vinylacetate and crotonic acid (INCI-name VA/Crotonates Copolymer, trade name Luvise™ CA66 available from BASF); copolymers of octylacrylamide, acrylic acid, butylamino methacrylate, methyl methacrylate and hydroxypropyl methacrylate (INCI-name Octylacrylamide/Acrylates/Butylaminoethyl Methacrylate Copolymer, available from National Starch and Chemical Company); copolymer of alkylacrylate, acrylic acid and alkylacrylamide (INCI-name Acrylates/t-Butylacrylamide Copolymer; trade name Ultrahold™ 8 available from BASF).

### Optional ingredients

Gel forming or hair fixing polymers with acid groups are preferably neutralised up to 50 bis 100%. Nonlimiting examples of neutralising agents include primary or secondary organic amines, or inorganic bases such as ammonia, NaOH, KOH, ammonium hydroxide etc.. Preferred are amino alcohols with 1 to 10 carbon atoms and 1 to 3 hydroxy groups such as aminomethyl propanol (AMP), monethanolamine, diethanol amine, triethanolamine, tetrahydroxypropyl ethylendiamine, diisopropanolamine, tromethamine, and mixtures thereof.

The compositions of the invention can optionally contain a plasticizer for the first or second copolymer or for the additional hair fixing polymers. Any plasticizer suitable for use in hair care products or for topical application to the hair or skin can be used. A wide variety of plasticizers are known in the art. These include acetyl triethylcitrate, triethylcitrate, glycerin, diisobutyl adipate, butyl stearate, phtalates and propylene glycol. Plasticizers are typically used at levels of from about 0.01% to about 10%, by weight of the composition, preferably from about 0.05% to about 3%, more preferably from about 0.05% to about 1%.

The composition according to the invention can also contain conventional cosmetic additives usually used in hair treatment compositions, e.g. fragrances and perfume oils in an amount of 0.01 to 1% by weight; preservatives, such as parabene or iodopropynyl butylcarbamate in an amount of 0.01 to 1% by weight; moisturiser; opacifying agents such as ethylenglycol distearate, fatty acid monoalkylolamide in an amount of about 0.2 to 5% by weight; buffer substances, such as sodium citrate or sodium phosphate, in an amount of 0.1 to 1% by weight; hair care substances, such as e.g. betaine, panthenol, plant extracts, vegetable extracts, protein hydroylsates and silk hydrolysates, lanolin derivatives, in an amount of 0.1 to 5% by weight; physiologically compatible silicone derivatives, such as volatile or non-volatile silicone oils or high molecular weight siloxane polymers in an amount of 0.05 to 20% by weight; light protective agents, antioxidants, radical-trapping agents, anti-dandruff agents; fatty alcohols; vitamines; direct dye compounds; luster-imparting substances and combability-improving substances in an amount of 0.01 to 2% by weight; surfactants and emulsifying agents which can be nonionic, anionic, cationic or amphoteric such as ethoxylated fatty alcohols, fatty alcohol sulfates, alkylbenzene sulfonate, alkyl trimethylammonium salts, alkylbetaine in an amount of 0.1 to 15% by weight; product coloring agents in an amount of 0.1 to 1% by weight; pigments in an amount of 0.01 to 25% by weight.

### Rheology

The hair styling composition of the invention has preferably the form of a gel and the viscosity of the gel preferably amounts to at least 1000 mPa s, e.g. from 1000 to 100000 mPa s, especially preferably from 2000 to 50000 mPa s, and most preferably from 2500 to 15000 mPa s, measured as dynamic viscosity with a Haake VT-550 Rheometer, measurement body SV-DIN at a temperature of 25° C and at a shear rate of 50 s⁻¹. The composition according to the invention is preferably in the form of a clear, transparent or at least translucent gel but it can also be turbid or non-transparent due to a content of pigments, pearl-gloss agents or other undissolved substances. The gel can be colored or colorless.

### METHOD OF MAKING

The compositions of the present invention can be made by conventional formulation and mixing techniques.

### METHOD OF USE

The compositions of the present invention are used in conventional ways to provide the hair styling/holding benefits of the present invention, in particular for increasing at least one of humidity resistance, moisture resistance and sweat resistance of a hair style. Such method generally involves application of an effective amount of the product to dry, slightly damp, or wet hair before and/or after the hair is arranged to a desired style. The composition is then dried or allowed to dry. By "effective amount" is meant an amount sufficient to provide the hair hold and style benefits desired considering the length and texture of the hair. In general, from about 0.5g to about 50g of product will be applied to the hair, depending upon the particular product formulation, dispenser type, length of hair, and type of hair style.

### EXAMPLES

The compositions illustrated in the following examples illustrate specific embodiments of the hair styling compositions of the present invention, but are not intended to be limiting thereof. Other modifications can be undertaken by the skilled artisan without departing from the scope of this invention. These exemplified embodiments of the hair styling composition of the present invention provide styling and volumizing benefits with increased humidity resistance, moisture resistance or sweat resistance of the hair style. The compositions illustrated in the following examples are prepared by conventional formulation and mixing methods. All exemplified amounts are listed as weight percents and exclude minor materials such as diluents, preservatives, color solutions, imagery ingredients, botanicals, and so forth, unless otherwise specified. If a trade name is mentioned as ingredient and the respective product is itself a mixture (e.g. a solution, emulsion, dispersion etc.), then the exemplified amount relates to this mixture.

### Test methods

Tests have been performed for assessing the moisture resistance against water and for assessing the removability of hair styling gel compositions. Standard test hair strands of equal hair quality and of 1 g each are treated with 0,3 g of the respective test composition. The treated hair strands are dried for one hour at room temperature (25°C). The strands are dipped into water for subsequent periods of from 1 to 3 seconds. After each period the fixing effect was assessed. The fixing effect was defined to be lost when the hair strand bends down more than 45° when held horizontaly. The procedure of dipping into water and assessing the fixing effect was repeated until the fixing effect was lost. The "water resistance" is the maximum of accumulated dipping time before the fixing effect is lost. Initial dipping times are 3 seconds. Water resistances above 10 seconds were measured with dipping times of less than 3 seconds for higher accuracy of the test results. Afterwards the removability is tested by washing the hair strands with a conventional shampoo.

### Example 1 A-D

| | 1A | 1B | 1C | ID |
|---|---|---|---|---|
| Luviset Clear™ 1 | 4.00 g | 20.00 g | - | - |
| Acudyne™ 180² | 5.00 g | - | 8.25 g | - |
| Fixate™ Plus³ | 6.70 g | - | - | 13.30 g |
| Neutraliser/pH | 2.72 g | - | 0.80 g | 2.20 g |
| Ethanol | 20.00 g | 30.00 g | 15.00 g | 30.00 g |
| Water | Ad 100 g | Ad 100 g | Ad 100 g | Ad 100 g |
| Water resistance | 16 sec | < 1sec | 3 sec | 6 sec |

| | | | | |
|---|---|---|---|---|
| ¹ INCI-name VP/Methacrylamide/Vinyl Imidazole Copolymer, available from BASF; 20% in water ² INCI-name Acrylates/Hydroxyesters Acrylates Copolymer, available from Rohm and Haas Company; 49% in water ³ INCI-name Polyacrylate-14, available from Noveon, Inc.; 30% in water | | | | |

### Example 1.1 A-D

| | 1.1 A | 1.1 B | 1.1 C | 1.1 D |
|---|---|---|---|---|
| Luviset Clear™¹ | 4.00 g | 20.00 g | - | - |
| Acudyne™ 180² | 5.00 g | - | 8.25 g | - |
| Fixate™ Plus ³ | 6.70 g | - | - | 13.30 g |
| Neutraliser/pH | 2.72 g | - | 0.80 g | 2.20 g |
| Ethanol | - | - | - | - |
| Water | Ad 100 g | Ad 100 g | Ad 100 g | Ad 100 g |
| Water resistance | 12 sec | < 1sec | < 1 sec | < 1 sec |

Compositions 1A and 1.1A according to the invention shows a synergistically improved water resistance of the styling effect. All compositions can be easily removed by shampooing.

### Example 2 water resistant hair styling gel

| | |
|---|---|
| 1,0 | Carbomer (Carbopol™ 980) |
| 1,8 | Aminomethylpropanol (95%) |
| 6,0 | Fixate™ Plus (Polyacrylate -14, 30% in water) |
| 5,0 | Acudyne™ 180 (Acrylates/Hydroxyester Acrylates Copolymer, 49% in water) |
| 2,0 | Glycerol |
| 0,3 | Fragrance |
| 20,0 | Ethanol |
| Ad 100 | Water |

### Example 3 water resistant hair styling gel

| | |
|---|---|
| 0,8 | Acrylates/C10-30 Alkyl Acrylate Crosspolymer (Carbopol™ 1382) |
| 1,6 | Aminomethylpropanol (95%) |
| 5,0 | Fixate™ Plus (Polyacrylate -14, 30% in water) |
| 6,0 | Acudyne™ 180 (Acrylates/Hydroxyester Acrylates Copolymer, 49% in water) |
| 1 | Polyvinylpyrrolidone |
| 1,0 | Propyleneglycol |
| 0,4 | Fragrance, color |
| 10,0 | Ethanol |
| Ad 100 | Water |

### Example 4 water resistant hair styling gel

| | |
|---|---|
| 0,8 | Carbomer (Carbopol™ 980) |
| 1,6 | Aminomethylpropanol (95%) |
| 7,0 | Fixate™ Plus (Polyacrylate -14, 30% in water) |
| 7,0 | Acudyne™ 180 (Acrylates/Hydroxyester Acrylates Copolymer, 49% in water) |
| 4 | Luviset Clear™ (VP/Methacrylamide/Vinyl Imidazole Copolymer, 20% in water) |
| 1,0 | Glycerol |
| 0,4 | Fragrance, color |
| Ad 100 | Water |

### Example 5 water resistant hair styling gel

| | |
|---|---|
| 1 | Xanthan Gum (Keltrol T™) |
| 1 | Aminomethylpropanol (95%) |
| 3,0 | Fixate™ Plus (Polyacrylate -14, 30% in water) |
| 8,0 | Acudyne™ 180 (Acrylates/Hydroxyester Acrylates Copolymer, 49% in water) |
| 1 | Vinylpyrrolidone/vinylacetate copolymer |
| 4 | Propyleneglycol |
| 0,3 | Fragrance |
| Ad 100 | Water |

### Example 6 water resistant hair styling gel

| | |
|---|---|
| 1 | Hydroxyethylcellulose (Natrosol™) |
| 1 | Aminomethylpropanol (95%) |
| 5,0 | Fixate™ Plus (Polyacrylate -14, 30% in water) |
| 6,0 | Acudyne™ 180 (Acrylates/Hydroxyester Acrylates Copolymer, 49% in water) |
| 2 | Glycerol |
| 0,3 | Fragrance |
| Ad 100 | Water |

### Example 7 water resistant hair styling gel

| | |
|---|---|
| 0,8 | Acrylates/Beheneth-25 Methacrylate Copolymer (Aculyne™ 28) |
| 1,5 | Aminomethylpropanol (95%) |
| 7,0 | Fixate™ Plus (Polyacrylate -14, 30% in water) |
| 3,0 | Acudyne™ 180 (Acrylates/Hydroxyester Acrylates Copolymer, 49% in water) |
| 1 | Acrylates/t-Butylacrylamide Copolymer (Ultrahold™ 8) |
| 3,0 | Propyleneglycol |
| 0,3 | Fragrance |
| 20,0 | Ethanol |
| Ad 100 | Water |

### Example 8 water resistant hair styling gel

| | |
|---|---|
| 0,8 | Ammonium Acryldimethyltaurate/VP Copolymer (Aristoflex™ AVC) |
| 1,4 | Aminomethylpropanol (95%) |
| 6,0 | Fixate™ Plus (Polyacrylate -14, 30% in water) |
| 5,0 | Acudyne™ 180 (Acrylates/Hydroxyester Acrylates Copolymer, 49% in water) |
| 2,5 | Glycerol |
| 0,3 | Fragrance |
| 20 | Ethanol |
| Ad 100 | Water |

Conventional hair styling gels are getting tacky at high air humidity (e.g. relative humidities up to 95%) or in a drizzle rain and the hair style of hair treated with conventional hair styling gels will tend to collapse under these conditions. Hair styled with compositions of examples 2 to 8 withstand high humidity and drizzle better.

## Claims

1. A hair styling composition comprising in an aqueous or aqueous-alcoholic carrier
(A) at least one first copolymer selected from copolymers of
(a1) at least one acidic vinyl monomer, selected from acrylic acid and methacrylic acid, or a salt thereof,
(a2) at least one hydrophobic nonionic vinyl monomer, selected from acrylic acid esters and methacrylic acid esters,
(a3) at least one first associative monomer and
(a4) at least one monomer selected from the group consisting of a second associative monomer different from the first associative monomer, a semihydrophobic monomer, and a combination thereof;
wherein the associative monomers have (i) an ethylenically unsaturated end group, (ii) a hydrophilic midsection and (iii) a hydrophobic or semi-hydrophobic end group;
(B) at least one second copolymer selected from copolymers of
(b1) at least one C3 to C8 monoethylenically unsaturated monocarboxylic acid monomer,
(b2) at least one nonionic vinyl monomer selected from acrylic acid alkyl esters and methacrylic acid alkyl esters, and
(b3) at least one hydroxy substituted nonionic vinyl monomer selected from acrylic acid hydroxyalkyl esters and methacrylic acid hydroxyalkyl esters.

2. The composition of claim 1 comprising from 0,1 to 15 % by weight of first copolymer (A) and from 0,1 to 15 % by weight of second copolymer (B).

3. The composition of any preceding claim wherein the nonionic vinyl monomers (a2) are selected from acrylic acid C1- to C4-alkyl esters and methacrylic acid C1- to C4-alkyl esters; and wherein the monomer (a4) is an associative monomer differing from (a3) in the hydrophobic end groups which are idependently selected from C8- to C40-hydrocarbon end groups.

4. The composition of any preceding claim wherein the monomer mixture includes at least one semihydrophobic monomer (a4) which is selected from monomers having one of the following formulas
R¹CH=CR¹-A-(CH₂)ₚ-(O)ᵣ-(R³O)ᵥ-R⁴
or
D-A-(CH₂)ₚ-(O)ᵣ-(R³O)ᵥ-R⁴
wherein, in each of the formulas, each R¹ is independently H, C₁-C₃₀ alkyl, -C(O)OH, or -C(O)OR²; R² is C₁-C₃₀ alkyl; A is -CH₂C(O)O- , -C(O)O-, -O-, -CH₂O-, -NHC(O)NH-, -C(O)NH-, -Ar-(CE₂)_{z}-NHC(O)O-, -Ar-(CE₂)_{z}-NHC(O)NH-, or -CH₂CH₂NHC(O)-; Ar is a divalent aryl; E is H or methyl; z is 0 or 1; p is an integer in the range of 0 to about 30, and r is 0 or 1, with the proviso that when p is 0, r is 0, and when p is in the range of 1 to about 30, r is 1; (R₃O)ᵥ is a polyoxyalkylene, which is a homopolymer, a random copolymer or a block copolymer of C₂-C₄ oxyalkylene units, wherein R³ is C₂H₄, C₃H₆, C₄H₈, and v is an integer in the range of about 5 to about 250, R⁴ is H or C₁-C₄ alkyl; and D is a C₈-C₃₀ unsaturated alkyl or a carboxy-substituted C₈-C₃₀ unsaturated alkyl.

5. The composition of claim 4 wherein the monomer mixture includes at least one semihydrophobic monomer (a4) which is selected from monomers having one of the following formulas
CH₂=CH-O-(CH₂)ₐ-O-(C₃H₆O)_{b}-(C₂H₄O)_{c}-H
or
CH₂=H-CH₂-O-(C₃H₆O)_{d}-(C₂H₄O)ₑ-H;
wherein a is 2, 3, or 4; b is an integer in the range of 1 to about 10; c is an integer in the range of about 5 to about 50; d is an integer in the range of 1 to about 10; and e is an integer in the range of about 5 to about 50.

6. The composition of any preceding claim wherein the first copolymer (A) is made from at least one additional compound selected from the group consisting of crosslinking agents, chain transfer agents, and a combination thereof.

7. The composition of any preceding claim wherein the monoethylenically unsaturated monocarboxylic acid monomer (b1) is selected from acrylic acid and methacrylic acid; and wherein the nonionic vinyl monomers (b2) are comprising at least one first monomer selected from methacrylic acid C1 to C3 alkyl esters and at least one second monomer selected from acrylic acid C2 to C5 alkyl esters; and wherein the hydroxy substituted nonionic vinyl monomer (b3) is selected from methacrylic acid C1 to C5 hydroxyalkyl esters and acrylic acid C1 to C5 hydroxyalkyl esters.

8. The composition of any preceding claim wherein the monoethylenically unsaturated monocarboxylic acid monomer (b1) is methacrylic acid; and wherein the nonionic vinyl monomers (b2) are comprising a first monomer which is methyl methacrylate and a second monomer which is n-butyl acrylate; and wherein the hydroxy substituted nonionic vinyl monomer (b3) is hydroxyethyl methacrylate.

9. The composition of any preceding claim comprising from 1 to 25% by weight ethanol.

10. The composition of any preceding claim additionally comprising at least one gel forming agent.

11. The composition according to claim 10 wherein the gel forming agent is a gel forming polymer selected from copolymers of at least one first monomer selected of acrylic acid and methacrylic acid and at least one second monomer selected of esters of acrylic acid and ethoxylated fatty alcohols; crosslinked polyacrylic acid; copolymers of vinyl pyrrolidone and ammonium acryloyl dimethyltaurate; hydroxyalkyl cellulose; hydroxyalkyl guar; xanthan; dehydroxanthan.

12. The composition of any preceding claim additionally comprising at least one polyhydric alcohol selected from glycerol and C2- to C4-alkylen glycols.

13. The composition of any preceding claim comprising at least one additional hair fixing polymer.

14. The composition according to claim 13 wherein the additional hair fixing polymer is selected from polyvinylpyrrolidone; copolymers of vinylpyyrolidone and vinylacetate; copolymers of vinylpyyrolidone, methacrylamide and vinylimidazole; copolymers of vinylacetate and crotonic acid; copolymers of octylacrylamide, acrylic acid, butylamino methacrylate, methyl methacrylate and hydroxypropylmethacrylate; copolymer of alkylacrylate, acrylic acid and alkylacrylamide.

15. The composition of any preceding claim wherein the composition has the form of a gel with a viscosity of at least 1000 mPa s at 25°C and at a shear rate of 50 s⁻¹.

16. Use of a composition according to any preceding claim for increasing at least one of humidity resistance, moisture resistance and sweat resistance of a hair style.

## Patentansprüche

1. Frisierzusammensetzung, umfassend in einem wässrigen oder wässrig-alkoholischen Träger
(A) mindestens ein erstes Copolymer, ausgewählt aus Copolymeren von
(a1) mindestens einem sauren Vinylmonomer, ausgewählt aus Acrylsäure und Methacrylsäure oder einem Salz davon,
(a2) mindestens einem hydrophoben nichtionischen Vinylmonomer, ausgewählt aus Acrylsäureestern und Methacrylsäureestern,
(a3) mindestens einem ersten assoziativen Monomer und
(a4) mindestens einem Monomer, das ausgewählt ist aus der Gruppe bestehend aus einem zweiten assoziativen Monomer, das sich von dem ersten assoziativen Monomer unterscheidet, einem semihydrophoben Monomer und einer Kombination davon;
wobei die assoziativen Monomere (i) eine ethylenisch ungesättigte Endgruppe, (ii) einen hydrophilen Mittelabschnitt und (iii) eine hydrophobe oder semihydrophobe Endgruppe aufweisen;
(B) mindestens ein zweites Copolymer, ausgewählt aus Copolymeren von
(b1) mindestens einem monoethylenisch ungesättigten C3- bis C8-Monocarbonsäuremonomer,
(b2) mindestens einem nichtionischen Vinylmonomer, ausgewählt aus Acrylsäurealkylestern und Methacrylsäurealkylestern, und
(b3) mindestens einem hydroxysubstituierten nichtionischen Vinylmonomer, ausgewählt aus Acrylsäurehydroxyalkylestern und Methacrylsäurehydroxyalkylestern.

2. Zusammensetzung nach Anspruch 1, umfassend von 0,1 bis 15 Gew.-% das erste Copolymer (A) und von 0,1 bis 15 Gew.-% das zweite Copolymer (B).

3. Zusammensetzung nach einem der vorstehenden Ansprüche, wobei die nichtionischen Vinylmonomere (a2) ausgewählt sind aus Acrylsäure-C1-C4-alkylestern und Methacrylsäure-C1-C4-alkylestern und wobei das Monomer (a4) ein assoziatives Monomer ist, das sich von (a3) in den hydrophoben Endgruppen unterscheidet, die unabhängig voneinander aus C8- bis C40-Koh-lenwasserstoffendgruppen ausgewählt sind.

4. Zusammensetzung nach einem der vorstehenden Ansprüche, wobei die Monomermischung mindestens ein semihydrophobes Monomer (a4) umfasst, das ausgewählt ist aus Monomeren mit einer der folgenden Formeln
R¹CH=CR¹-A-(CH₂)ₚ-(O)ᵣ-(R³O)ᵥ-R⁴
oder
D-A-(CH₂)ₚ-(O)ᵣ-(R³O)ᵥ-R⁴
wobei in jeder der Formeln jedes R¹ unabhängig H, C₁-C₃₀-Alkyl, -C(O)OH oder -C(O)OR² ist; R² C₁-C₃₀-Alkyl ist; A -CH₂C(O)O-, -C(O)O-, -O-, -CH₂O-, -NHC(O)NH-, -C(O)NH-, -Ar-(CE₂)_{z}-NHC(O)O-, -Ar-(CE₂)_{z}-NHC(O)NH- oder -CH₂CH₂NHC(O)- ist; Ar ein zweiwertiges Aryl ist; E H oder Methyl ist; z 0 oder 1 ist; p eine ganze Zahl im Bereich von 0 bis etwa 30 ist und r 0 oder 1 ist, mit der Maßgabe, dass, wenn p 0 ist, r 0 ist, und wenn p im Bereich von 1 bis etwa 30 liegt, r 1 ist; (R³O)ᵥ ein Polyoxyalkylen ist, welches ein Homopolymer, ein statistisches Copolymer oder ein Blockcopolymer von C₂-C₄-Oxyalkyleneinheiten ist, worin R³ C₂H₄, C₃H₆, C₄H₈ ist und v eine ganze Zahl im Bereich von etwa 5 bis etwa 250 ist, R⁴ H oder C₁-C₄-Alkyl ist; und D ein ungesättigtes C₈-C₃₀-Alkyl oder ein carboxysubstituiertes ungesättigtes C₈-C₃₀-Alkyl ist.

5. Zusammensetzung nach Anspruch 4, wobei die Monomermischung mindestens ein semihydrophobes Monomer (a4) umfasst, das ausgewählt ist aus Monomeren mit einer der folgenden Formeln
CH₂=CH-O-(CH₂)ₐ-O-(C₃H₆O)_{b}-(C₂H₄O)_{c}-H
oder
CH₂=CH-CH₂-O-(C₃H₆O)_{d}-(C₂H₄O)ₑ-H;
worin a 2, 3 oder 4 ist; b eine ganze Zahl im Bereich von 1 bis etwa 10 ist; c eine ganze Zahl im Bereich von etwa 5 bis etwa 50 ist; d eine ganze Zahl im Bereich von 1 bis etwa 10 ist; und e eine ganze Zahl im Bereich von etwa 5 bis etwa 50 ist.

6. Zusammensetzung nach einem der vorstehenden Ansprüche, wobei das erste Copolymer (A) aus mindestens einer zusätzlichen Verbindung hergestellt ist, die ausgewählt ist aus der Gruppe bestehend aus Vernetzungsmitteln, Kettenübertragungsmitteln und einer Kombination davon.

7. Zusammensetzung nach einem der vorstehenden Ansprüche, wobei das monoethylenisch ungesättigte Monocarbonsäuremonomer (b1) aus Acrylsäure und Methacrylsäure ausgewählt ist und wobei die nichtionischen Vinylmonomere (b2) mindestens ein erstes Monomer umfassen, das aus Methacrylsäure-C1-C3-alkyl-estern ausgewählt ist, und mindestens ein zweites Monomer, das aus Acrylsäure-C2-C5-alkylestern ausgewählt ist, und wobei das hydroxysubstituierte nichtionische Vinylmonomer (b3) aus Methacrylsäure-C1-C5-hydroxyalkylestern und Acrylsäure-C1-C5-hydroxyalkylestern ausgewählt ist.

8. Zusammensetzung nach einem der vorstehenden Ansprüche, wobei das monoethylenisch ungesättigte Monocarbonsäuremonomer (b1) Methacrylsäure ist und wobei die nichtionischen Vinylmonomere (b2) ein erstes Monomer umfassen, das Methylmethacrylat ist, und ein zweites Monomer, das n-Butylacrylat ist, und wobei das hydroxysubstituierte nichtionische Vinylmonomer (b3) Hydroxyethylmethacrylat ist.

9. Zusammensetzung nach einem der vorstehenden Ansprüche, umfassend von 1 bis 25 Gew.-% Ethanol.

10. Zusammensetzung nach einem der vorstehenden Ansprüche; zusätzlich umfassend mindestens ein Gelierungsmittel.

11. Zusammensetzung nach Anspruch 10, wobei das Gelierungsmittel ein Gelierungspolymer ist, das ausgewählt ist aus Copolymeren von mindestens einem ersten Monomer, ausgewählt aus Acrylsäure und Methacrylsäure, und mindestens einem zweiten Monomer, ausgewählt aus Estern von Acrylsäure und ethoxylierten Fettalkoholen; vernetzter Polyacrylsäure; Copolymeren von Vinylpyrrolidon und Ammoniumacryloyldimethyltaurat; Hydroxyalkylcellulose; Hydroxyalkylguar; Xanthan; Dehydroxanthan.

12. Zusammensetzung nach einem der vorstehenden Ansprüche, zusätzlich umfassend mindestens ein Polyol, ausgewählt aus Glycerol und C2- bis C4-A1-kylenglykolen.

13. Zusammensetzung nach einem der vorstehenden Ansprüche, umfassend mindestens ein zusätzliches Haarfestigungspolymer.

14. Zusammensetzung nach Anspruch 13, wobei das zusätzliche Haarfestigungspolymer ausgewählt ist aus Polyvinylpyrrolidon; Copolymeren von Vinylpyrrolidon und Vinylacetat; Copolymeren von Vinylpyrrolidon, Methacrylamid und Vinylimidazol; Copolymeren von Vinylacetat und Krotonsäure; Copolymeren von Octylacrylamid, Acrylsäure, Butylaminomethacrylat, Methylmethacrylat und Hydroxypropylmethacrylat; Copolymer von Alkylacrylat, Acrylsäure und Alkylacrylamid.

15. Zusammensetzung nach einem der vorstehenden Ansprüche, wobei die Zusammensetzung bei 25 °C und einer Scherrate von 50 s⁻¹ die Form eines Gels mit einer Viskosität von mindestens 1000 mPa.s aufweist.

16. Verwendung einer Zusammensetzung nach einem der vorstehenden Ansprüche zur Steigerung mindestens einer der Eigenschaften Feuchtigkeitsbeständigkeit, Nässebeständigkeit und Schweißbeständigkeit einer Frisur.

## Revendications

1. Composition de coiffage des cheveux comprenant, dans un véhicule aqueux ou aqueux-alcoolique
(A) au moins un premier copolymère choisi parmi les copolymères
(a1) d'au moins un monomère vinylique acide, choisi parmi l'acide acrylique et l'acide méthacrylique ou un de leurs sels,
(a2) d'au moins un monomère vinylique non ionique hydrophobe, choisi parmi les esters de l'acide acrylique et les esters de l'acide méthacrylique,
(a3) d'au moins un premier monomère associatif et
(a4) d'au moins un monomère choisi dans le groupe constitué d'un deuxième monomère associatif différent du premier monomère associatif, un monomère semi-hydrophobe et une de leurs combinaisons ;
où les monomères associatifs présentent (i) un groupe terminal éthyléniquement insaturé, (ii) une section centrale hydrophile et (iii) un groupe terminal hydrophobe
ou semi-hydrophobe ;
(B) au moins un deuxième copolymère choisi parmi les copolymères
(b1) d'au moins un monomère acide monocarboxylique en C3 à C8 monoéthyléniquement insaturé,
(b2) d'au moins un monomère vinylique non ionique, choisi parmi les esters alkyliques de l'acide acrylique et les esters alkyliques de l'acide méthacrylique, et
(b3) d'au moins un monomère vinylique non ionique substitué par hydroxy, choisi parmi les esters hydroxyalkyliques de l'acide acrylique et les esters hydroxyalkyliques de l'acide méthacrylique.

2. Composition selon la revendication 1, comprenant 0,1 à 15 % en poids d'un premier copolymère (A) et 0,1 à 15 % en poids d'un deuxième copolymère (B).

3. Composition selon l'une quelconque des revendications précédentes, dans laquelle les monomères vinyliques non ioniques (a2) sont choisis parmi les esters alkyliques en C1 à C4 de l'acide acrylique et les esters alkyliques en C1 à C4 de l'acide méthacrylique et dans laquelle le monomère (a4) est un monomère associatif différent de (a3) au niveau des groupes terminaux hydrophobes qui sont indépendamment choisis parmi des groupes terminaux hydrocarbonés en C8 à C40.

4. Composition selon l'une quelconque des revendications précédentes, dans laquelle le mélange de monomères comprend au moins un monomère semi-hydrophobe (a4) qui est choisi parmi les monomères présentant une des formules suivantes
R¹CH=CR¹-A-(CH₂)ₚ-(O)ᵣ-(R³O)ᵥ-R⁴
ou
D-A-(CH₂)ₚ-(O)ᵣ-(R³O)ᵥ-R⁴
où, dans chacune des formules, chaque R¹ représente, indépendamment H, un alkyle en C₁-C₃₀, -C(O)OH, ou -C(O)OR² ; R² représente un alkyle en C₁-C₃₀ ; A représente -CH₂C(O)O-, -C(O)O-, -O-, -CH₂O-, -NHC(O)NH-, -C(O)NH-, -Ar-(CE₂)_{z}-NHC(O)O-, -Ar-(CE₂)_{z}-NHC(O)NH-, ou -CH₂CH₂NHC(O)-; Ar représente un groupe aryle divalent ; E représente H ou méthyle ; z est 0 ou 1 ; p représente un entier dans la plage de 0 à environ 30, et r est 0 ou 1, à condition que, lorsque p est 0, r soit 0, et lorsque p est dans la plage de 1 à environ 30, r soit 1 ; (R³O)ᵥ représente un polyoxyalkylène, qui est un homopolymère, un copolymère aléatoire ou un copolymère séquencé d'unités oxyalkylène en C₂-C₄, où R³ représente C₂H₄, C₃H₆, C₄H₈, et v représente un entier dans la plage d'environ 5 à environ 250, R⁴ représente H ou un alkyle en C₁-C₄ ; et D représente un alkyle en C₈-C₃₀ insaturé ou un alkyle en C₈-C₃₀ insaturé substitué par carboxy.

5. Composition selon la revendication 4, dans laquelle le mélange de monomères comprend au moins un monomère semi-hydrophobe (a4) qui est choisi parmi les monomères présentant l'une des formules suivantes
CH₂=CH-O-(CH₂)ₐ-O-(C₃H₆O)_{b}-(C₂H₄O)_{c}-H
ou
CH₂=CH-CH₂-O-(C₃H₆O)_{d}-(C₂H₄O)ₑ-H ;
dans lesquelles a est 2, 3, ou 4 ; b est un entier dans la plage de 1 à environ 10 ; c est un entier dans la plage d'environ 5 à environ 50 ; d est un entier dans la plage de 1 à environ 10 ; et e est un entier dans la plage d'environ 5 à environ 50.

6. Composition selon l'une quelconque des revendications précédentes, dans laquelle le premier copolymère (A) est préparé à partir d'au moins un composé supplémentaire choisi dans le groupe constitué d'agents de réticulation, agents de transfert de chaîne, et une de leurs combinaisons.

7. Composition selon l'une quelconque des revendications précédentes, dans laquelle le monomère acide monocarboxylique monoéthyléniquement insaturé (b1) est choisi parmi l'acide acrylique et l'acide méthacrylique ; et dans laquelle les monomères vinyliques non ioniques (b2) comprennent au moins un premier monomère choisi parmi les esters alkyliques en C1 à C3 de l'acide méthacrylique et au moins un deuxième monomère choisi parmi les esters alkyliques en C2 à C5 de l'acide acrylique ; et dans laquelle le monomère vinylique non ionique substitué par hydroxy (b3) est choisi parmi les esters hydroxyalkyliques en C1 à C5 de l'acide méthacrylique et les esters hydroxyalkyliques en C1 à C5 de l'acide acrylique.

8. Composition selon l'une quelconque des revendications précédentes, dans laquelle le monomère acide monocarboxylique monoéthyléniquement insaturé (b1) est l'acide méthacrylique ; et dans laquelle les monomères vinyliques non ioniques (b2) comprennent un premier monomère qui est le méthacrylate de méthyle et un deuxième monomère qui est l'acrylate de n-butyle ; et dans laquelle le monomère vinylique non ionique substitué par hydroxy (b3) est le méthacrylate d'hydroxyéthyle.

9. Composition selon l'une quelconque des revendications précédentes, comprenant de 1 à 25 % en poids d'éthanol.

10. Composition selon l'une quelconque des revendications précédentes, comprenant, en outre, au moins un agent gélifiant.

11. Composition selon la revendication 10, dans laquelle l'agent gélifiant est un polymère gélifiant choisi parmi les copolymères d'au moins un premier monomère choisi parmi l'acide acrylique et l'acide méthacrylique et d'au moins un deuxième monomère choisi parmi les esters de l'acide acrylique et les alcools gras éthoxylés ; le poly(acide acrylique) réticulé ; les copolymères de vinylpyrrolidone et d'acryloyldiméthyltaurate d'ammonium ; l'hydroxyalkylcellulose ; l'hydroxyalkylguar ; le xanthane ; le déshydroxanthane.

12. Composition selon l'une quelconque des revendications précédentes, comprenant, en outre, au moins un alcool polyhydrique choisi parmi le glycérol et les alkylèneglycols en C2 à C4.

13. Composition selon l'une quelconque des revendications précédentes, comprenant au moins un polymère supplémentaire fixant les cheveux.

14. Composition selon la revendication 13, dans laquelle le polymère supplémentaire fixant les cheveux est choisi parmi la polyvinylpyrrolidone ; les copolymères de vinylpyrrolidone et d'acétate de vinyle ; les copolymères de vinylpyrrolidone, de méthacrylamide et de vinylimidazole ; les copolymères d'acétate de vinyle et d'acide crotonique ; les copolymères d'octylacrylamide, d'acide acrylique, d'aminométhacrylate de butyle, de méthacrylate de méthyle et de méthacrylate d'hydroxypropyle ; le copolymère d'acrylate d'alkyle, d'acide acrylique et d'alkylacrylamide.

15. Composition selon l'une quelconque des revendications précédentes, où la composition est sous forme d'un gel présentant une viscosité d'au moins 1000 mPa.s à 25 °C et à une vitesse de cisaillement de 50 s⁻¹.

16. Utilisation d'une composition selon l'une quelconque des revendications précédentes, pour augmenter au moins l'une parmi la résistance à l'humidité, la résistance à la buée et la résistance à la transpiration d'une coiffure.
